# EUROPEAN PATENT SPECIFICATION

(11) **EP 4 221 699 B1**
(45) Date of publication and mention of the grant of the patent: **04.12.2024**
(21) Application number: 21798444.2
(22) Date of filing: 01.10.2021
(51) Int. Cl.: A61K 31/166, A61K 31/167, A61K 31/17, A61K 31/404, A61K 31/4406, A61K 31/455, A61K 31/513, A61K 31/7088, A61K 45/06, A61P 31/20, A61P 35/00

(54) **PHARMACEUTICAL COMPOSITION COMPRISING EX527 AND CISPLATIN OR DOXORUBICIN FOR USE IN PREVENTING AND/OR TREATING AN HPV INDUCED TUMOUR**
PHARMAZEUTISCHE ZUSAMMENSETZUNG ENTHALTEND EX527 UND CISPLATIN ODER DOXORUBICIN ZUR VERWENDUNG BEI DER VORBEUGUNG UND/ODER BEHANDLUNG EINES HPV-INDUZIERTEN TUMORS
COMPOSITION PHARMACEUTIQUE COMPRENANT DE L'EX527 ET DU CISPLATINE OU DE LA DOXORUBICINE DESTINÉE À ÊTRE UTILISÉE DANS LA PRÉVENTION ET/OU LE TRAITEMENT D'UNE TUMEUR INDUITE PAR LE HPV

(30) Priority: 02.10.2020 IT 202000023281
(43) Date of publication of application: 09.08.2023
(60) Divisional application: 24201272.2
(73) Proprietor: Università degli Studi del Piemonte Orientale "Amedeo Avogadro", 13100 Vercelli (IT)
(72) Inventor: GARIGLIO, Marisa, 28100 Novara (IT); LO CIGNO, Irene, 28100 Novara (IT); CALATI, Federica, 28100 Novara (IT)
(74) Representative: Studio Torta S.p.A.
(86) International application number: PCT/IB2021/059055
(87) International publication number: WO 2022/070161

(56) References cited:
- US-A1- 2013 338 178
- US-A1- 2018 200 233
- SINGH SAPNA ET AL: "Expression/localization patterns of sirtuins (SIRT1, SIRT2, and SIRT7) during progression of cervical cancer and effects of sirtuin inhibitors on growth of cervical cancer cells", TUMOR BIOLOGY, KARGER, BASEL, CH, vol. 36, no. 8, 21 March 2015 (2015-03-21), pages 6159 - 6171, XP036218515, ISSN: 1010-4283, [retrieved on 20150321], DOI: 10.1007/S13277-015-3300-Y
- VELEZ-PEREZ ANNELIESE ET AL: "SIRT1 overexpression in cervical squamous intraepithelial lesions and invasive squamous cell carcinoma", HUMAN PATHOLOGY, SAUNDERS, PHILADELPHIA, PA, US, vol. 59, 6 October 2016 (2016-10-06), pages 102 - 107, XP029849261, ISSN: 0046-8177, DOI: 10.1016/J.HUMPATH.2016.09.019

## Description

### Technical Field

The present invention relates to a pharmaceutical composition comprising EX527 for use in preventing and/or treating a human papilloma virus (HPV) induced tumour, wherein the pharmaceutical composition further comprises doxorubicin or cisplatin.

The invention is defined in the claims. Any subject-matter which is not encompassed by the claims is not part of the invention and provided for reference or comparison purposes only.

Any reference to a non-patentable method of treatment of the human or animal body by therapy involving a compound or composition is to be understood as referring to said compound or composition for use in said method.

### Background of the Invention

Human papillomaviruses (HPV) are viruses that are widely spread in the population that infect the multilayered squamous epithelia present in the genital, cutaneous and upper respiratory tract. The Papillomaviridae family now has more than 200 human genotypes divided into 5 genera (alpha to nu) based on their taxonomy.

High neoplastic risk HPVs, including HPV16 and HPV18, are connected with the development of tumours of the anogenital and head and neck regions. HPV-induced carcinogenesis depends on the ability of viral E6 and E7 proteins to manipulate a number of cellular pathways, including those involved in the innate immune response. The oncogenic potential of these two viral proteins is certainly based on their ability to inhibit the activity of cellular oncosuppressors and specifically E6 binds p53, degrading it, while E7 binds and inactivates the pRb protein. The viral protein E7 is also able to upregulate another cellular protein with deacetylase function, called SIRT1; this increased expression is evident in HPV-induced tumours, such as cervical cancer. SIRT1 is involved in the regulation of several aspects of the life cycle of high oncogenic risk HPVs. US 2013/338178 describes the use of SIRT1 or SIRT2 inhibitor in the inhibition of the viral production in cells infected with a virus.

SIRT1 also plays a fundamental role in the functional regulation of various cellular proteins, including the tumour suppressor protein p53 which is deacetylated by SIRT1 promoting the degradation thereof.

A SIRT1 antagonist, EX527, is a specific pharmacological inhibitor of this enzyme and has been proposed for the treatment of neurodegenerative Huntington's disease.

SINGH SAPNA ET AL ("Expression/localization patterns of sirtuins (SIRT1, SIRT2, and SIRT7) during progression of cervical cancer and effects of sirtuin inhibitors on growth of cervical cancer cells",TUMOR BIOLOGY,vol. 36, no. 8, 21 March 2015, pages 6159-6171) discloses an activity of the SIRT1 inhibitor EX527 in the induction of cytostasis of cervical cancer cell lines.

VELEZ-PEREZ ANNELIESE ET AL ("SIRT1 overexpression in cervical squamous intraepithelial lesions and invasive squamous cell carcinoma", HUMAN PATHOLOGY, vol. 59, 6 October 2016, pages 102-107) cite SINGH SAPNA ET AL. and independently confirm the disclosure.

US 2013/338178 A1 (SHENK THOMAS [US] ET AL) relates to methods of inhibiting virus production comprising contacting a virus-infected cell with a SIRT1 inhibitor and a SIRT2 inhibitor. The claimed methods include the treatment of HPV and can encompmass contacting the virus with a second agent. In a particular embodiment, the cells in question can be tumor cells.

US 2018/200233 A1 discloses the co-administration of spinacine and doxorubicin in the treatment or prevention of a cancer caused by a human papilloma virus.

To date, the treatment of cervical cancer and of the tumours of the head-neck region is highly invasive and with devastating effects on patients as it involves radiotherapy, chemotherapy or surgery. For this reason, an alternative anti-tumour therapy with reduced side effects could have great potential.

There is therefore a need in the art to provide new molecules for preventing and/or treating a human papilloma virus (HPV) induced tumours.

### Disclosure of Invention

It is an object of the present invention to provide a composition capable of preventing and/or treating a human papilloma virus (HPV) induced tumour.

According to the present invention, this object is achieved by the composition for use according to Claim 1.

### Brief description of the figures

For a better understanding of the present invention, it will also be described with reference to the accompanying figures, which show the following:
- Figure 1 represents an SDS-page of protein extracts obtained from cells treated with the pharmacological inhibitor EX527 (or with the solvent, DMSO) for 48 h or genetically silenced for SIRT1 (or control siRNA, siCTRL) for 72 h decorated with anti-SIRT1, anti-p53, anti-E6 (HPV16 and HPV18), anti-E7 (HPV16 and HPV18), and anti-α-tubulin antibodies;
- Figure 2 represents an SDS-page of protein extracts obtained from cells pre-treated with the pharmacological inhibitor EX527 (or with the solvent, DMSO) for 48 h and incubated with the proteosome inhibitor MG132 for 8 h decorated with anti-E6 (HPV16 and HPV18), anti-E7 (HPV16 and HPV18), and anti-α-tubulin antibodies;
- Figure 3 represents a clonogenic assay of HeLa, CaSki, NOKE6E7, C33A and HNO150 cells treated with the pharmacological inhibitor EX527 (or the solvent, DMSO) for 15 days;
- Figure 4 represents a graph of cell viability following treatment with the pharmacological inhibitor EX527 (or with the solvent, DMSO) in combination with doxorubicin (DX) at 16, 40, 64 h;
- Figure 5 represents a graph of the cell cycle phases wherein cells are pre-treated with the pharmacological inhibitor EX527 (or with the solvent, DMSO) for 48 h and incubated with doxorubicin (DX) for 16 h;
- Figure 6 represents the percentage of apoptotic cells following treatment with the pharmacological inhibitor EX527 (or with the solvent, DMSO) for 48 h and incubated with doxorubicin (DX) for 24 h;
- Figure 7 represents the cell viability of NOKE6E7 cells following treatment with the pharmacological inhibitor EX527 (or with the solvent, DMSO) in combination with cisplatin (CIS) at 24, 48, 72 h (A), a graph of the cell cycle phases (B) and the percentage of apoptotic cells (C) of NOKE6E7 cells pre-treated with the pharmacological inhibitor EX527 (or with the solvent, DMSO) for 48 h and incubated with cisplatin (CIS) for 24 h;
- Figure 8 represents the cell count (A), cell viability (B), a graph of the cell cycle phases (C) and the percentage of apoptotic cells (D) of NOKE6E7 cells pre-treated with the pharmacological inhibitor EX527 (or with the solvent, DMSO) for 24 h and then irradiated with 2 Gy;
- Figure 9 represents a graph of the preclinical experimental model of C57BL/6J mice wherein C3 tumour cells were inoculated and treated with the pharmacological inhibitor EX527 (or with the solvent, DMSO) for a fortnight every two days (A), a graph with the trend over time of the volumes of the developed tumour mass (B), immunohistochemical images in which sections of tumours removed from mice treated with EX527 (or with the solvent, DMSO) were decorated with anti-p53 and anti-Ki67 (C).

### Detailed description of the invention

According to the present invention a pharmaceutical composition comprising a SIRT1 inhibitor or antagonist, namely EX527, for use in preventing and/or treating a human papilloma virus (HPV) induced tumour, wherein the pharmaceutical composition further comprises a chemotherapeutic drug, namely doxorubicin or cisplatin.

Preferably, the inhibitor or antagonist induces the degradation of the E6 and/or E7 HPV proteins through the proteasome.

By the term "inhibitor" or "enzymatic inhibitor" of SIRT1 there is intended a molecule capable of establishing a chemical bond with an enzyme, thereby decreasing its activity. The inhibitor interferes with the catalytic activity of the enzyme, for example by preventing the substrate from entering the enzyme's active site.

By the term SIRT1 "antagonist" there is intended a molecule which, although selectively binding to a receptor, does not activate it, blocking signal transduction. In other words, the antagonists are endowed with a specific affinity for a given receptor, but they lack intrinsic efficacy, that is, they are not able to cause the effects that the bond between the receptor and its specific molecule would activate. The term "antagonism" generically refers to an interaction that leads to a loss of effect of a substance.

Preferably, the pharmaceutical composition is used in combination with radiotherapy.

Preferably, the pharmaceutical composition is in the form of immediate release tablets from 80 to 120 mg, preferably 100 mg.

Even more preferably, the pharmaceutical composition in the form of the aforesaid tablets is used in a dosage of one tablet/day.

The following examples show that by inhibiting SIRT1 in cells infected and transformed by HPV by means of a SIRT1 inhibitor or antagonist such as EX527 it is possible to inhibit the expression of the oncogene proteins E6 and E7, restore the functional expression of p53 and more generally block cell proliferation. While the role of SIRT1 in inhibiting viral replication in HPV-infected cells was already known, it was not known or foreseeable that SIRT1 inhibition would inhibit the proliferation of tumour cells already transformed by the virus.

### Examples

### Example 1

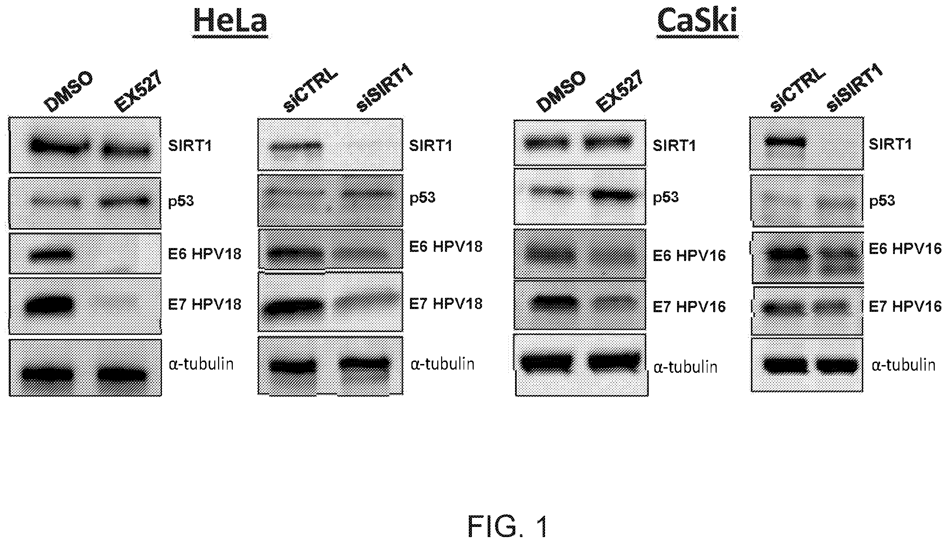

This example, the results of which are shown in Figure 1, demonstrates that SIRT1 inhibition in cells transformed by HPV16 or HPV18 results in an increased expression of p53 and concomitant reduction in the expression of the oncogenic viral E6 and E7 proteins.

In order to understand the antitumour/antiviral nature of SIRT1 inhibitors in HPV-induced tumours, the effect of the pharmacological inhibitor EX527 (SIGMA ALDRICH) and gene silencing of SIRT1 was analysed by using siRNA (small interfering RNA) on the expression levels of proapoptotic molecules such as p53 and of the viral E6 and E7 oncoproteins. To do so, HeLa or CaSki cells (ATCC - LGC STANDARDS), transformed by integration of the HPV18 and HPV16 viral genome respectively, were treated for 48 h with EX527 at a concentration of 80 µM or equal volume of solvent, dimethyl sulfoxide (DMSO, SIGMA ALDRICH) or transfected with siRNAs against SIRT1 (L-003540-00-0005, ON-TARGETplus SMARTpool siRNA, Dharmacon) or with a control siRNA for 72 h using Lipofectamine RNAiMAX (Invitrogen) as a transfection reagent. After this incubation period at 37 °C, the expression levels of p53, E6 and E7 proteins were tested in order to assess the effect of SIRT1 inhibition. The α-tubulin protein was included as an internal loading control. The cells were lysed with RIPA buffer (50 mM Tris-HCl pH 7.4; 150 mM NaCl; 1 mM EDTA; 1% Nonidet P-40; 0.1% SDS; 0.50 deoxycholate; protease inhibitors) for 30 min on ice. The samples were subsequently centrifuged for 10 minutes at 13000 rpm at 4 °C to remove cellular debris and then the proteins were quantified using the Bradford method (BIO-RAD). 30 µg of protein extract were taken, to which the appropriate amount of Laemmli sample buffer (50 mM Tris-HCl pH 6.8, 2% SDS, 10% glycerol, 2% β-mercaptoethanol) was added. The thus obtained solution was heated to 95 °C for 5 minutes and separated by polyacrylamide (12%)-SDS page gel electrophoresis. At the end of the run, the gel was transferred onto a nitrocellulose membrane which was subsequently hybridised with antibodies directed against the proteins of interest (primary antibody) and then with a secondary antibody. The membrane was then developed using the chemiluminescence method (Pierce ECL Western Blotting Substrate, ThermoFisher Scientific) and analysed using the ChemiDoc instrument (BIORAD). From the results obtained, it can be observed that the pharmacological or gene inhibition of SIRT1 results in an increase in the proapoptotic protein p53 and at the same time in a reduction in the expression of the oncogenic viral E6 and E7 proteins. Considering that the tumour phenotype of HPV-transformed cells is totally dependent on maintaining the expression of oncogenic proteins that in turn maintain low levels of p53, the condition created by SIRT1 inhibition creates the conditions for reversion of the tumour phenotype induced by HPV16 or 18 and potentially other HPV genotypes. Similar experiments were repeated on NOKE6E7 cells (immortalised human keratinocytes stably expressing E6 and E7 of HPV16) obtaining similar results, using as HPV-negative control cells the lines C33A derived from an HPV-negative cervical cancer and HNO150 derived from a HPV-negative squamous cell laryngeal carcinoma where the same results were not obtained.

### Example 2

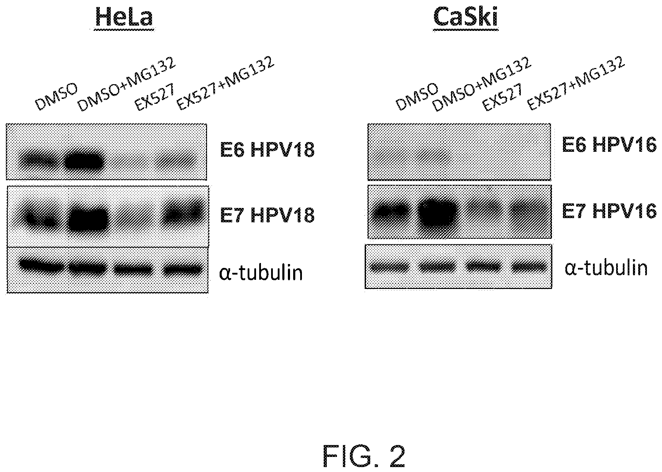

This example, the results of which are shown in Figure 2, demonstrates how the increased degradation of the oncogenic viral E6 and E7 proteins induced by SIRT1 inhibition occurs via proteasome.

In order to determine whether the inhibition of E6 or E7 proteins was due to a transcriptional or post-translational modification, HeLa or CaSki cells, pre-treated for 48 h with EX527 at a concentration of 80 µM or equal volume of solvent, dimethyl sulfoxide (DMSO, SIGMA ALDRICH), were incubated with a proteosome inhibitor, MG132 (SIGMA ALDRICH) for 8 h at a concentration of 30 µM. After this incubation period at 37 °C, the expression levels of viral E6 and E7 proteins were tested. The α-tubulin protein was included as an internal loading control. The cells were lysed with RIPA buffer (50 mM Tris-HCl pH 7.4; 150 mM NaCl; 1 mM EDTA; 1% Nonidet P-40; 0.1% SDS; 0.5% deoxycholate; protease inhibitors) for 30 min on ice. The samples were subsequently centrifuged for 10 minutes at 13000 rpm at 4 °C to remove cellular debris and then the proteins were quantified using the Bradford method (BIO-RAD). 30 µg of protein extract were taken, to which the appropriate amount of Laemmli sample buffer (50 mM Tris-HCl pH 6.8, 2% SDS, 10% glycerol, 2% β-mercaptoethanol) was added. The thus obtained solution was heated to 95 °C for 5 minutes and separated by polyacrylamide (12%)-SDS page gel electrophoresis. At the end of the run, the gel was transferred onto a nitrocellulose membrane which was subsequently hybridised with antibodies directed against the proteins of interest (primary antibody) and then with a secondary antibody. The membrane was then developed using the chemiluminescence method (Pierce ECL Western Blotting Substrate, ThermoFisher Scientific) and analysed using the ChemiDoc instrument (BIORAD). From the results obtained, it can be observed that already basally E6 and E7 are partly degraded via proteosome, since in cells treated with the solvent DMSO and MG132 there is a recovery in the expression of the tested proteins compared to the control cells (DMSO); in the cells in which SIRT1 was inhibited and MG132 added, there is a restoration of their expression. This data indicates that the reduction in the expression levels of the viral E6 and E7 oncoproteins consequent to the pharmacological inhibition of SIRT1 occurs through increased degradation of the proteins themselves via proteosome and is not due to a reduced gene transcription.

### Example 3

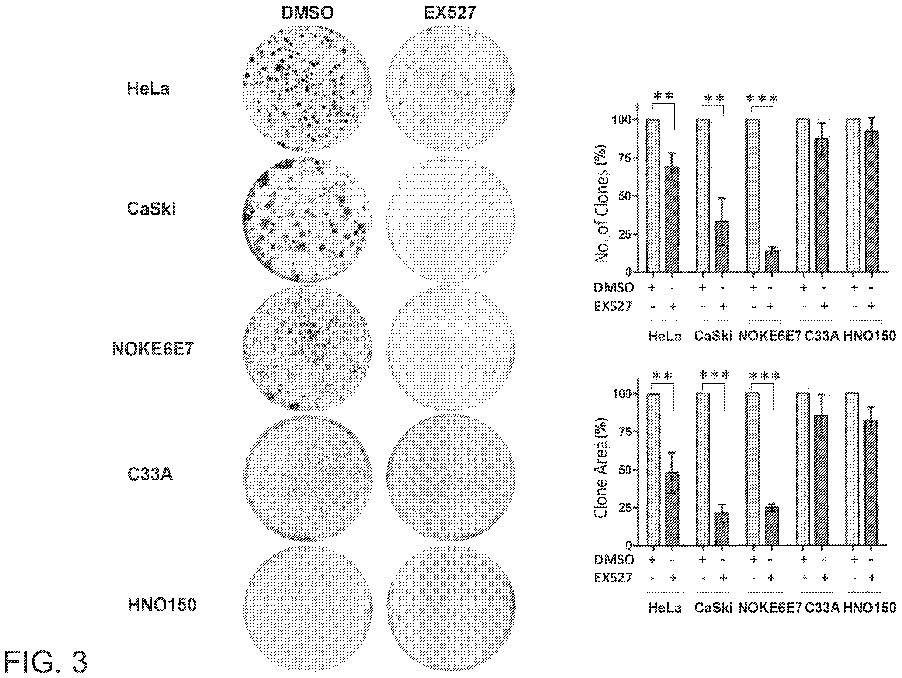

Considering the role of p53 in the control of cell proliferation, the effect of the pharmacological inhibitor EX527 on the ability of the cells transformed by HPV to form clones was analysed. For this reason, HeLa, CaSki, NOKE6E7, C33A, and HNO150 cells were low density plated and treated with EX527 at a concentration of 80 µM or equal volume of DMSO solvent for 15 days. The culture medium containing EX527 was changed every 3 days. After 15 days of treatment, the formed colonies were washed with PBS1X (Sigma-Aldrich) and fixed with 4% paraformaldehyde (PAF, Sigma-Aldrich) for 10 minutes. The colonies were then washed with PBS1X (Sigma-Aldrich) and stained with 0.1% (w/v) crystal violet for 10 minutes. Finally, the colonies were washed with deionised water, acquired and counted using the Cytation1 instrument (BioTek). As it can be observed in Figure 3, pharmacological inhibition of SIRT1 reduces the clonogenic capacity of the HPV-positive lines, but not of the HPV-negative lines.

### Example 4

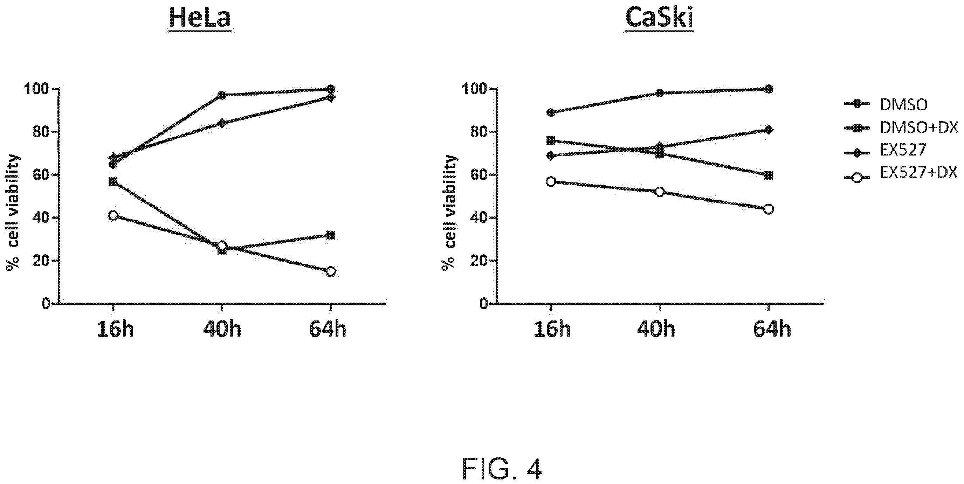

This example, the results of which are shown in Figure 4, demonstrates how SIRT1 inhibition makes HPV-transformed cells more sensitive to the antiproliferative activity of doxorubicin.

The observations described above prompted an analysis of whether HPV-positive cells in which SIRT1 is inhibited with consequent reduced expression of E6 and E7 and restoration of p53, could be more sensitive to the treatment with genotoxic drugs, such as doxorubicin (DX, SIGMA ALDRICH). Firstly, cell viability was determined following treatment with EX527 in combination with doxorubicin against HeLa and CaSki cells, using the MTT (3-(4,5-dimethylthiazol-2-yl)-2,5-diphenyltetrazolium bromide; SIGMA- ALDRICH) colorimetric assay. To this purpose, the cells were exposed to a concentration of EX527 of 80 µM or equal volume of solvent, dimethyl sulfoxide (DMSO, SIGMA ALDRICH) for 24 h, after this period elapsed the cells were treated with DX (1.5 µM for HeLa, 0.5 µM for CaSki) for a further 16, 40, 64 h. Subsequently, the supernatant was removed, the well was washed with PBS1X (SIGMA-ALDRICH) and incubated for 2 hours at 37 °C and 5% of CO₂ with DMEM medium supplemented with 500 µg/mL MTT solution. At the end of the incubation, the supernatant was again removed and the cells solubilised in DMSO. On these solubilised cells, absorbance was measured at a wavelength of 570 nm, using the Victor3 multiplate reader spectrophotometer (Perkin Elmer). The absorbance value obtained from the sample treated with the carrier alone at the maximum dose was considered equal to 100% viability and the other values were related thereto. The results obtained indicate that cell proliferation is inhibited by EX527 (red line) particularly at 40 h after treatment in HeLa and at 40 and 64 h in CaSki, and the mortality rate increases more after treatment with DX with a significant increase in the cells pretreated with EX527 (yellow line).

### Example 5

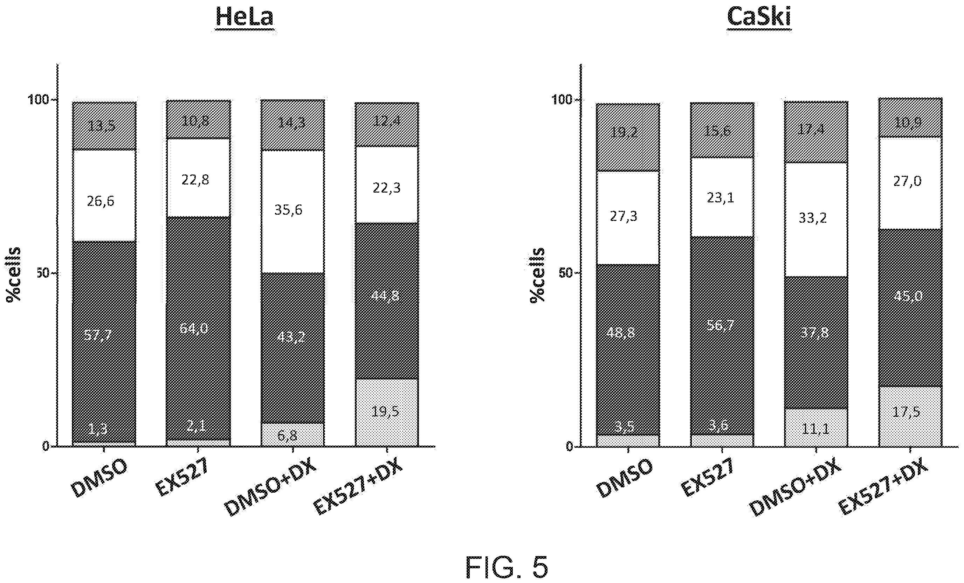

This example, the results of which are shown in Figure 5, demonstrates that SIRT1 inhibition in HPV-transformed cells results in cell cycle arrest in the G0/G1 phase with increase in cell death (sub-G0/G1 phase).

Having established that the combined EX527+DX treatment significantly reduces cell proliferation or viability, we assessed the impact of this combined treatment on the cell cycle in HeLa and CaSki cells. For this purpose, HeLa and CaSki cells were treated with EX527 (80uM) for 24 h, or with an equal volume of DMSO solvent and after this period elapsed, they were treated with DX (1.5 µM for HeLa, 0.5 µM for CaSki) for a further 16 h. After this incubation period at 37°C, the cells were labelled with propidium iodide (IP). Propidium iodide (3,8-diamino-5-diethylmethyl aminopropyl-6-phenylphenanthridine diiodide) (IP) is a synthetic dye characterised by low fluorescence (red-orange), capable of selectively binding to nucleic acids. It is known that the DNA content varies according to the phase of the cycle in which a cell is found. In particular, the G2 phase and mitosis (M) have twice as much DNA as the G1 phase, while during the DNA synthesis phase (S phase), the cell has an intermediate amount of DNA between the content in G1 and G2. Since the apoptotic cells, once resuspended in an appropriate buffer, release small DNA fragments, a reduction in IP fluorescence (related to the decrease in DNA content) can be observed in them. In this way it is possible to assess the percentage of apoptotic cells that are placed in the so-called hypodiploid peak as well as the percentage distribution of the cells in the various phases of the cell cycle: G0/G1, S and G2/M. For IP staining to be performed it is necessary to obtain a lysis of the cytoplasmic membrane to allow the penetration of the dye inside the cell. The cells are then fixed in cold 70% ethanol for at least 60 min at -20 °C, washed in phosphate buffered saline (PBS), incubated for 15 min at 37 °C with 20 µg/mL of IP, 100 ug/mL of RNAase type XII-A in a citrate solution (0.71 g Na₂HPO₄, 0.735 g C₆H₅Na₃O₇ · 2H₂O, 100 µL Triton X-100, pH 7.8). The use of RNase is necessary to eliminate the double helix RNA that might bind IP and thus interfere with the specific DNA staining. The cytofluorimetric analysis was performed on the FACSCalibur flow cytometer instrument (BD Biosciences) (excitation at 488 nm and emission 400 nm) and the results were analysed with the FlowJo programme. As shown in the Figure, a cell cycle arrest in the G0/G1 phase is observed in HeLa and CaSki cells following treatment with EX527, as well as an increase in cell death (sub-G0/G1 phase) after treatment with DX which results significantly increased in the cells pretreated with EX527. These data confirm that SIRT1 inhibition makes HPV-transformed cells more sensitive to death induced by treatment with genotoxic drugs such as DX.

### Example 6

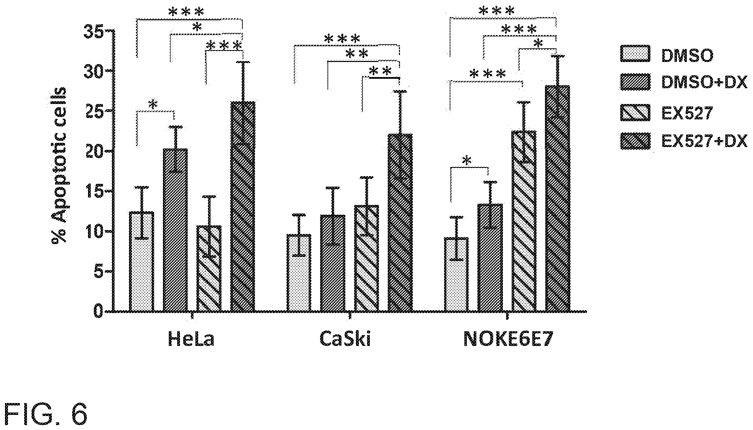

This example, the results of which are shown in Figure 6, demonstrates how SIRT1 inhibition in HPV-transformed cells results in an increase in the number of apoptotic cells following combined EX527 treatment and doxorubicin.

Having observed the increase in the sub-G0/G1 phase following treatment with EX527 and DX, the percentage of apoptotic cells following this treatment was assessed. For this purpose, HeLa, CaSki, and NOKE6E7 cells were treated with EX527 (80 µM) for 24 h, or with an equal volume of DMSO solvent and after this period elapsed, they were treated with DX (1.5 µM for HeLa and NOKE6E7, 0.5 µM for CaSki) for a further 24 h. After this incubation period at 37 °C, the cells were processed using the "Annexin V-FITC Apoptosis Detection Kit" (BioLegend), a technique that allows the presence of apoptotic and necrotic cells to be detected by cytofluorimetric analysis. This assay exploits the ability of the annexin protein to bind to phosphatidylserine (PS) and thus give fluorescence in green thanks to the conjugated fluorophore (FITC, fluorescein isothiocyanate). PS is a phospholipid normally found on the inner side of the cell membrane. During apoptosis processes, on the other hand, PS is exposed to the outside and allows annexin V to bind to it. As this bond can also occur during necrosis, following disruption of the plasma membrane, the propidium iodide (IP) fluorophore is added, which intersperses stoichiometrically and emits in the red if the cell membrane is not intact, so as to discriminate between living cells (annexin V and negative IP), apoptotic cells (annexin V positive, negative IP) and necrotic cells (annexin V negative, positive IP). Specifically, the cells were washed with PBS1X and then resuspended in Annexin V Binding Buffer. The cells were then incubated with FITC-annexin V (5 µl) and propidium iodide (IP) (10 µl) for 15 min at room temperature. Subsequently, 400 µl of Annexin V Binding Buffer were added to each tube and the cells were analysed on the FACSCalibur flow cytometer instrument (BD Biosciences) (excitation at 488 nm, emission 518 nm and 400 nm for FITC and IP respectively). The results were then analysed with the FlowJo programme. As shown in Figure 6, there is an increase in the percentage of apoptotic cells following treatment with DX, which results to have strongly increased in cells pre-treated with EX527. These data confirm that SIRT1 inhibition promotes the apoptosis of HPV-transformed cell lines, making them more sensitive to death induced by treatment with genotoxic drugs such as DX.

### Example 7

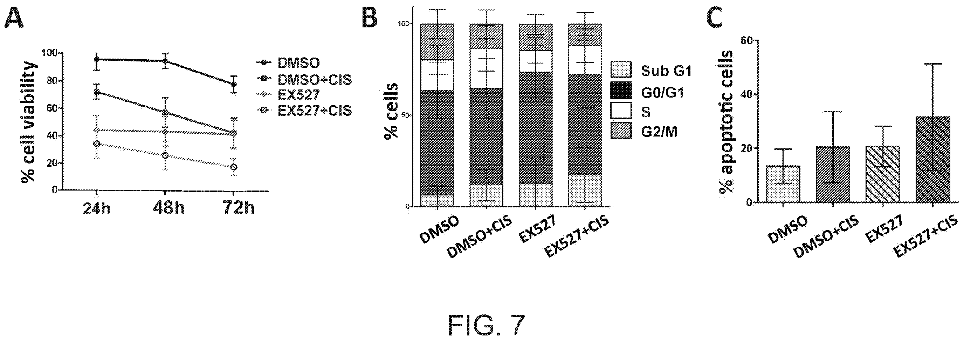

This example, the results of which are shown in Figure 7, demonstrates how SIRT1 inhibition makes HPV-transformed cells more sensitive to treatment with other genotoxic drugs, such as cisplatin.

In order to increase the clinical impact of this research, doxorubicin was then replaced with cisplatin as the chemotherapeutic drug usually used in the treatment of HPV-associated tumours. Firstly, cell viability was determined following treatment with EX527 in combination with cisplatin (CIS, 2.5 µM) for 24, 48, 72 h against NOKE6E7 cells using the MTT colorimetric assay (3-(4,5-dimethylthiazol-2-yl)-2,5-diphenyltetrazolium bromide; SIGMA- ALDRICH). To this purpose, the supernatant was removed, the well was washed with PBS1X (Sigma-Aldrich) and incubated for 2 hours at 37 °C and 5% of CO2 with DMEM medium supplemented with 500 µg/mL MTT solution. At the end of the incubation, the supernatant was again removed and the cells solubilised in DMSO. On these solubilised cells, absorbance was measured at a wavelength of 570 nm, using the Victor3 multiplate reader spectrophotometer (Perkin Elmer). The highest absorbance value obtained from the sample treated with the carrier alone was considered to be equal to 100% viability and the other values were related to it. The results obtained indicate that the mortality rate increases more in cells pre-treated with EX527 and following treatment with CIS (green line) (Panel A). In addition, we assessed the impact of this combined treatment on the cycle (Panel B) and on cell death (Panel C) by cytofluorometer analysis, as described previously in examples 5 and 6. For this, NOKE6E7 cells were treated with EX527 (80 µM) for 24 h, or with an equal volume of DMSO solvent and subsequently with CIS (2.5 µM) for a further 24 h. The results obtained confirm those observed with DX, indicating that also cisplatin induces an increase in the number of apoptotic cells if preceded by the treatment with EX527. This pharmacological inhibitor therefore increases the sensitivity of HPV-transformed cells even to the treatment with CIS.

### Example 8

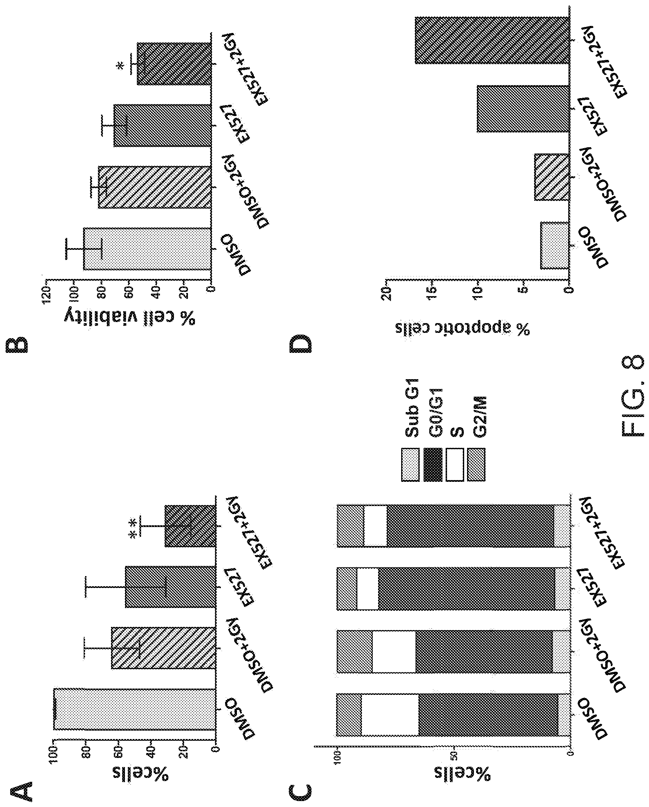

This example, the results of which are shown in Figure 8, demonstrates how SIRT1 inhibition increases the sensitivity of HPV-transformed cells to radiotherapy.

After having demonstrated that SIRT1 inhibition increases the sensitivity to chemotherapy, the next step was to assess whether the same effect could also be achieved for radiotherapy which is usually substituted or combined with chemotherapy. For this reason, NOKE6E7 cells were treated with EX527 (20 µM) for 24 h, or with an equal volume of DMSO solvent and then irradiated with 2 Gy at a dose of 600 cGy/min. 48 h after irradiation, the cells were washed with PBS1X (Sigma-Aldrich), stained with 0.1% crystal violet and incubated for 20 min at room temperature. Subsequently, the cells were washed with PBS1X and the crystal violet solubilised with 10% acetic acid and measured using a spectrophotometer at 595 nm (Figure 8A). In addition, cell viability was assessed using the MTT colorimetric assay, as previously described in examples 4 and 7 (Figure 8B). These results indicate that the treatment with EX527 reduces the number of cells and their viability, which further decrease following treatment with EX527 in combination with radiotherapy. Subsequently, the effect of EX527 together with radiotherapy on the cell cycle was evaluated, as described in example 5. As can be seen in Figure 8C, there is a cell cycle arrest in the G0/G1 phase following treatment with EX527, as well as an increase in cell death (sub-G0/G1 phase) following radiotherapy. Finally, cytofluorimetric analysis was carried out following staining with annexin V and IP, as described in example 6. Figure 8D shows a significant increase in the percentage of apoptotic cells in cells pre-treated with EX527 and subjected to radiotherapy. These results therefore confirm that SIRT1 inhibition increases cell death following exposure to a single dose of irradiation.

### Example 9

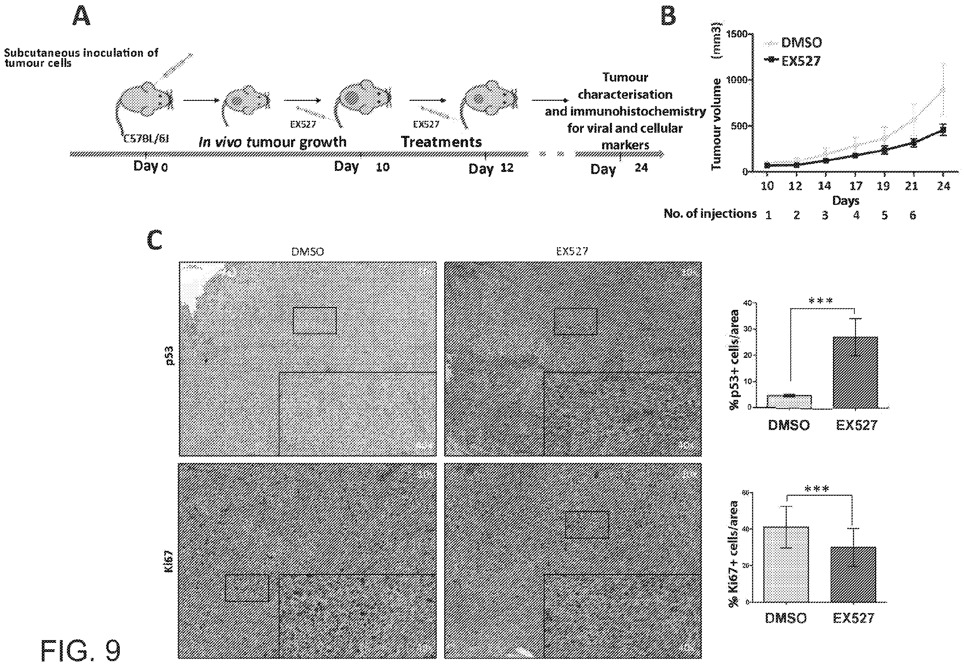

This example, the results of which are shown in Figure 9, demonstrates how SIRT1 inhibition reduces the tumourigenic activity of HPV16-transformed cells in a syngeneic mouse model of tumourigenesis in vivo.

On the basis of the results obtained in vitro which confirmed the antiproliferative activity of the pharmacological inhibitor of SIRT1, in vivo experiments were performed using a syngenic model of HPV-induced tumourigenesis involving subcutaneous inoculation of C57BL/6J mice with C3 tumour cells (containing the integrated HPV16 genome).

At time 0, 10^6 C3 cells are inoculated subcutaneously into the mouse, ten days after cell inoculation, the pharmacological SIRT1 inhibitor EX527 is inoculated intraperitoneally every two days for a fortnight at a concentration of 10 mg/kg or equal volume of DMSO solvent (Figure 9A). Before each inoculation, the developed tumours are measured using a caliper and at the end of two weeks are removed, weighed and processed for further histological investigation. The removed tumours were then fixed in formalin overnight and subsequently embedded in paraffin. The obtained blocks were cut using a microtome obtaining 5 um sections. These were then deparaffinised for 30 minutes in xylene and rehydrated using an ethanol scale with decreasing concentration. Subsequently, the sections were incubated with 3% peroxidase at room temperature for 8 minutes under stirring to eliminate endogenous peroxidase activity. Antigen unmasking was done by placing the slides in a pressure cooker with Buffer Citrate 10 mM pH 6 (Antigen Unmasking Solution, Vector Laboratories) for 15 minutes at 750W and 10 minutes at 300W. For immunohistochemistry analysis, the sections were first incubated with 10% horse serum (NHS, Vector Laboratories) to reduce the possibility of non-specific bonds and then with either the anti-p53 (MA5-15244; Thermo Fisher Scientific, diluted 1:1000) or anti-Ki67 (abl6667; abcam, diluted 1:200) primary antibody overnight at 4°C in a humid chamber. The following day, the sections were incubated with the biotinylated secondary antibody for 30 minutes at room temperature and then with RTU Vectastain ABC Reagent (Vector Laboratories) for 30 minutes at room temperature. Staining was carried out by incubation with diaminobenzidine tetrahydrochloride (DAB) for one or two minutes depending on the intensity of the dye developed and counter-staining with Mayer's haematoxylin. Finally, the sections were dehydrated using an ethanol scale with increasing concentration and xylene. The sections were then scanned by means of a digital scanner (Pannoramic MIDI, 3DHistech) and the positive cell count for the two markers was performed using the ImageJ software. The results obtained on 8 mice per treatment confirmed the ability of EX527 to inhibit tumour growth also in vivo. In fact, as reported in Figure 9, the treatment with the pharmacological inhibitor induces a significant reduction in the volume of the tumour mass compared to tumours treated with an equal volume of DMSO solvent (Figure 9B). Furthermore, the number of cells positive for p53 was higher in EX527-treated tumours than in control cells, while the number of cells positive for the proliferation marker Ki67 was significantly higher in control than in EX527-treated tumours (Figure 9C). These observations thus confirm the results obtained in vitro, indicating that EX527 also exhibits antiproliferative/anti-tumour activity in vivo. In addition, the increased expression of p53 in the neoplastic tissue implies a possible increased vulnerability of tumour cells to chemo-radiotherapy treatments, suggesting that it is possible to implement combined therapies EX527/Cisplatin/radiotherapy with reduced dosage of chemo-radiotherapy drugs and consequent reduction of the side effects connected with these therapies.

## Claims

1. A pharmaceutical composition comprising EX527 for use in preventing and/or treating a human papilloma virus (HPV) induced tumour, wherein the pharmaceutical composition further comprises doxorubicin or cisplatin.

2. The pharmaceutical composition for use according to claim 1, wherein the chemotherapeutic drug is cisplatin.

3. The pharmaceutical composition for use according to claim 1 or 2, wherein the pharmaceutical composition is used in combination with radiotherapy.

4. The pharmaceutical composition for use according to any of claims from 1 to 3, wherein the pharmaceutical composition is in the form of immediate release tablets from 80 to 120 mg.

5. The pharmaceutical composition for use according to claim 4, wherein the pharmaceutical composition is used in a dosage of one tablet/day.

## Patentansprüche

1. Pharmazeutische Zusammensetzung, umfassend EX527 zur Verwendung bei der Prävention und/oder Behandlung eines durch das humane Papillomavirus (HPV) induzierten Tumors, wobei die pharmazeutische Zusammensetzung ferner Doxorubicin oder Cisplatin umfasst.

2. Pharmazeutische Zusammensetzung zur Verwendung nach Anspruch 1, wobei das chemotherapeutische Arzneimittel Cisplatin ist.

3. Pharmazeutische Zusammensetzung zur Verwendung nach Anspruch 1 oder 2, wobei die pharmazeutische Zusammensetzung in Kombination mit Strahlentherapie verwendet wird.

4. Pharmazeutische Zusammensetzung zur Verwendung nach einem der Ansprüche 1 bis 3, wobei die pharmazeutische Zusammensetzung in Form von Tabletten mit sofortiger Freisetzung von 80 bis 120 mg vorliegt.

5. Pharmazeutische Zusammensetzung zur Verwendung nach Anspruch 4, wobei die pharmazeutische Zusammensetzung in einer Dosierung von einer Tablette/Tag verwendet wird.

## Revendications

1. Composition pharmaceutique comprenant EX527 pour une utilisation dans la prévention et/ou le traitement d'une tumeur induite par le papillomavirus humain (HPV), dans laquelle la composition pharmaceutique comprend en outre de la doxorubicine ou du cisplatine.

2. Composition pharmaceutique pour une utilisation selon la revendication 1, dans laquelle le médicament chimiothérapeutique est le cisplatine.

3. Composition pharmaceutique pour une utilisation selon la revendication 1 ou 2, dans laquelle la composition pharmaceutique est utilisée en association avec la radiothérapie.

4. Composition pharmaceutique pour une utilisation selon l'une quelconque des revendications 1 à 3, dans laquelle la composition pharmaceutique se présente sous la forme de comprimés à libération immédiate de 80 à 120 mg.

5. Composition pharmaceutique pour une utilisation selon la revendication 4, dans laquelle la composition pharmaceutique est utilisée à raison d'un comprimé par jour.
